# EUROPEAN PATENT SPECIFICATION

(11) **EP 2 142 067 B1**
(45) Date of publication and mention of the grant of the patent: **06.03.2013**
(21) Application number: 08744608.4
(22) Date of filing: 28.03.2008
(51) Int. Cl.: A47L 13/26, A47L 13/17

(54) **LIQUID APPLICATOR WITH AN ANGLED ELONGATED HEAD**
FLÜSSIGKEITSAPPLIKATOR MIT WINKELFÖRMIGEM VERLÄNGERTEM KOPF
APPLICATEUR DE LIQUIDE AYANT UNE TÊTE ALLONGÉE INCLINÉE

(30) Priority: 30.03.2007 US 909274 P
(43) Date of publication of application: 13.01.2010
(73) Proprietor: Allegiance Corporation, McGaw Park, Illinois 60085-6787 (US)
(72) Inventor: CHESAK, Eric, El Paso, Texas 79922 (US); BARDWELL, James, R., El Paso, TX 79912 (US); BALTEZOR, Michael, J., Lee's Summit, Missouri 64064 (US); LENZ, Aime, Kansas City, Missouri 64108 (US); CROSBY, Cynthia T., Bozeman, Montana 59718 (US)
(74) Representative: Iannone, Carlo Luigi
(86) International application number: PCT/US2008/058670
(87) International publication number: WO 2008/121827

(56) References cited:
- EP-A2- 0 339 260
- WO-A1-2005/099808
- WO-A2-2004/062709
- US-A- 3 981 304
- US-A- 4 507 111
- US-A1- 2006 039 741
- US-A1- 2006 039 742
- US-A1- 2006 039 742
- US-B1- 6 238 120
- US-B1- 6 595 940
- US-B2- 6 991 394

## Description

### BACKGROUND

Applicators for applying liquids such as medicaments or cleansing agents, are known in the prior art. Conventional applicators typically provide a generally cylindrical body construction with a glass ampoule, a sponge or tip secured to the head of the body, and a means for fracturing the ampoule. When the ampoule is fractured, the liquid stored therein is dispensed to the sponge for application.

Typically, the applicator sponge of conventional applicators has one flat side for applying liquid to a flat surface and a second flat side exposed to the internal body of the applicator. Due to the shape of the sponge, liquid can only be applied to one flat surface and cannot be used to apply liquid to multiple surfaces at one time. As such, it is difficult for these applicators to properly apply liquid to hard to reach places, such as between folds of a patient's skin.

WO-A-2004/062709 is directed to a liquid applicator for applying a desired liquid to a surface comprising a hollow body defining an internal chamber to receive at least two elongated ampoules formed of a frangible material and containing the liquid to be applied (see p. 3, line 25 to p. 4, line 2). The applicator comprises a lever projecting from body that flexes inward to fracture the ampoules substantially simultaneously when the lever is depressed towards the body. The applicator further comprises a porous element secured to the body of the applicator that closes off an open end of the body. When the ampoules are fractured, the released liquid saturates the porous element so that it can be applied to a surface.

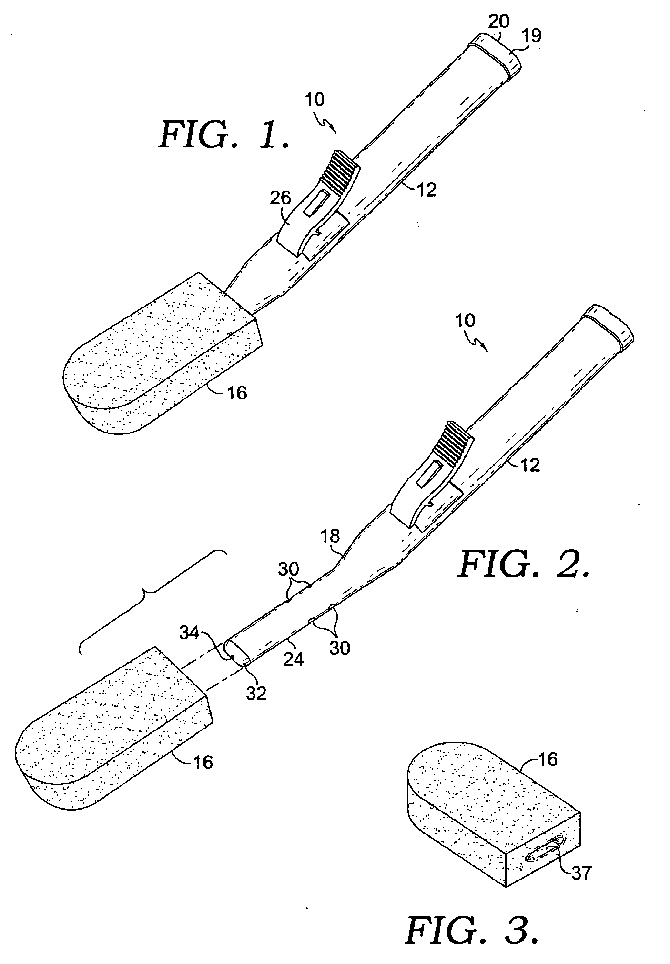

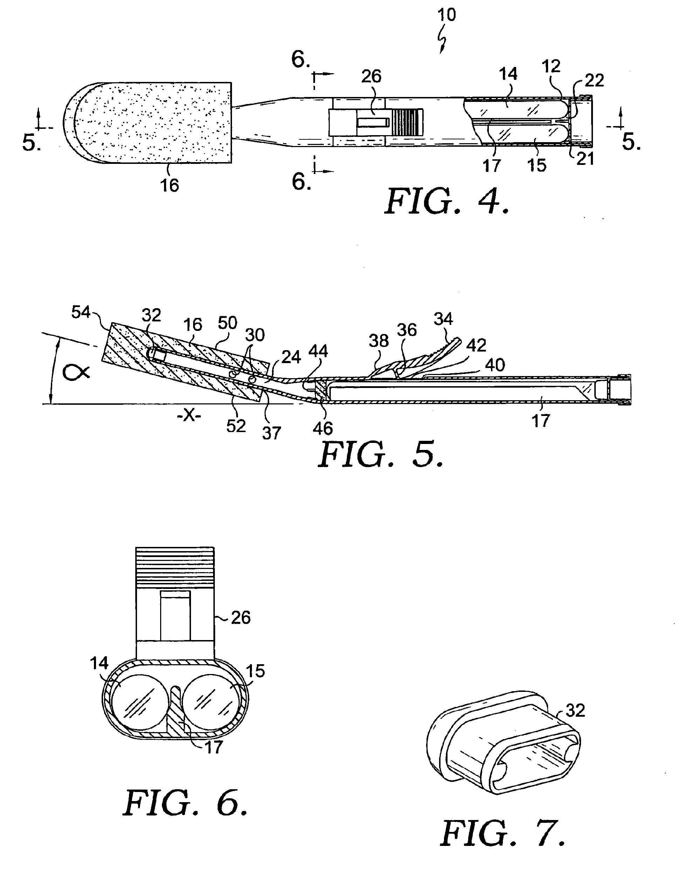

US-A-4507111 relates to liquid dispensers and applicators where a premeasured supply of liquid is disposed in an applicator handle and is selectively dispensed through the applicator. As shown in Figs. 1-5, D3 surgical scrub includes a sponge applicator 10, a tubular handle 11, and a cartridge 12 (see col. 4, lines 40-45). The cartridge member 12 is a generally tubular member that contains the fluid and has an outside periphery that matches the inside periphery of a portion of the tubular handle 11 (see col. 5, lines 5-30). The cartridge member 12 is made of a plastic material. A dispensing chamber 22 projects forward from the tubular handle 11 into the sponge applicator 10. The dispensing chamber provides free flow of pressurized fluid to the sponge 10 and provides rigidity to the sponge 10 (see col. 5, line 64 to col. 6, line 19). The dispensing chamber 22 includes a plurality of apertures 26 that are in fluid communication with the interior of the dispensing chamber 22 and the surrounding interior of the sponge applicator 10. A spike 23 in the form of a tube projects rearwardly from the dispensing chamber 22. The end of the spike 23 is tapered to a point so as to puncture the cartridge 18. In use, a user pushes down on the cartridge 12 to flex and permit the forward end 18 of the cartridge 12 to move forward and be ruptured by the point of the spike 23, as shown in Fig. 4 (see col. 6, lines 29-35).

In WO-A-2005/099808, a dispenser 410 includes an applicator 450 at one end, which is located in a sleeve 460 (see p. 26, lines 5-13). The sleeve 460 is preferably made of materials that are impermeable to the skin antiseptic composition located within the container 420. When the composition is delivered to the applicator 450 while the applicator 450 is located within the sleeve 460, any of the skin antiseptic composition that escapes from the applicator 450 is retained within the sleeve 460. WO-A-2005/099808 discloses that the sleeve 460 may be grasped by the user to pierce the seal and dispense the composition into the applicator and that the sleeve 460 can be removed to reveal the applicator.

### SUMMARY

In one embodiment, a liquid applicator for applying a desired liquid to a surface is provided. The liquid applicator comprises at least one ampoule formed of a frangible material and containing liquid to be applied and an elongated hollow body, said body defining an internal chamber adapted to receive said at least one ampoule. The applicator further comprises an elongated head projecting at an angle from said body, the elongated head having at least two openings for liquid to flow through. At least one mechanism projecting from said body flexes said body inwardly to fracture said at least one ampoule and a porous element is secured to the elongated head. Liquid flows through the at least two openings of the elongated head and through said porous element when said ampoule is fractured.

Additional aspects of the invention, together with the advantages and novel features appurtenant thereto, will be set forth in part in the description which follows, and in part will become apparent to those skilled in the art upon examination of the following, or may be learned from the practice of the invention. The objects and advantages of the invention may be realized and attained by means, instrumentalities and combinations particularly pointed out in the appended claims.

### BRIEF DESCRIPTION OF THE DRAWINGS

In the accompanying drawings which form a part of the specification and are to be read in conjunction therewith, and in which like reference numerals are employed to indicate like parts in the various views:

FIG. 1 is a perspective view of a liquid applicator constructed in accordance with an embodiment of the invention;

FIG. 2 is a perspective view of a liquid applicator with the porous element removed in accordance with an embodiment of the invention;

FIG. 3 is a perspective view of a porous element in accordance with an embodiment of the present invention;

FIG. 4 is a top plan view of a liquid applicator constructed in accordance with an embodiment of the invention with a portion of the applicator body removed to expose the inside of the body and the ampoules;

FIG. 5 is a side plan view of a liquid applicator constructed in accordance with an embodiment of the invention with a portion of the applicator body removed to expose the support feature and the angle of the elongated head from the body of the applicator;

FIG. 6 is a fragmentary cross-section view taken generally across line 6-6 of FIG. 4 in accordance with an embodiment of the present invention;

FIG. 7 is a perspective view of a tip cap removed from the elongated head in accordance with an embodiment of the present invention; and

FIG. 8 is a fragmentary cross-section view of a liquid applicator in accordance with an embodiment of the present invention.

### DETAILED DESCRIPTION

A liquid applicator for applying a desired liquid to a surface is described. The applicator comprises a hollow body defining an internal chamber to receive at least one elongated ampoules formed of a frangible material and containing the liquid to be applied. The liquid applicator further comprises at least one mechanism, for flexing said body inwardly to fracture the at least one ampoule when the at least one mechanism is depressed toward the body. The liquid applicator further comprises an elongated head projecting from the body. The elongated head as at least two openings for liquid to flow through when the at least one ampoule is fractured. A porous element is secured to the elongated head and covers all sides of the elongated head.

In use, after the ampoules are fractured, the liquid from the ampoules fills the body of the applicator and flows into the elongated head of the applicator. The liquid then passes through the at least two openings in the elongated head and saturates the porous element. The porous element may then be used to apply liquid to hard to reach places, such as between and under folds of skin. The liquid applicator may be utilized to prep obese persons for surgery and for vaginal and perineum-perirectal applications. The applicator allows access to areas, such as into cracks and crevices, where current applicators cannot reach.

With reference FIG. 1, FIG. 2, FIG. 4 and FIG. 5 in particular, where like reference numerals identify like elements in the various views, an embodiment of the liquid applicator is illustrated and designated generally by the numeral 10. Liquid applicator 10 generally includes a body 12, and a porous element 16 secured to elongated head 24 and at least one fracturing mechanism 26.

Two ampoules 14 and 15 are received in body 12. Ampoules 14 and 15 may be used for containing various liquids or gels such as medicaments, cleansing agents, cosmetics, polishes or the like. In the illustrated embodiment, ampoules 14 and 15 contain antiseptic solution to be applied to a patient's skin prior to surgery. Antiseptics that may be employed include chlorhexidine, olanexidine, alexidine and octenidine and salts thereof. The chlorhexidine salt used in preferred embodiments is chlorhexidine gluconate (CHG). CHG may be used with a variety of solvents, including water is the primary dissolving solvent or isopropyl alcohol as the primary dissolving solvent.

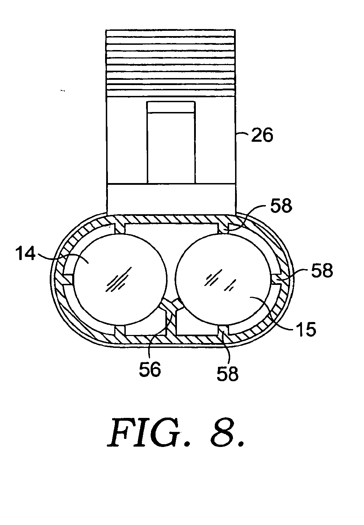

Ampoules 14 and 15 are illustrated as elongated cylinders each with a central longitudinal axis. In this embodiment, the liquid applicator 10 is constructed to house two 13 ml ampoules. The thickness of the walls of the 13 ml ampoules is 0.3 mm. It will be appreciated that the applicator may be constructed to house any size ampoules, including, but not limited to, two 20 ml ampoules having a wall thickness of 0.3 mm (as shown in FIG. 8) or two 6.5 ml ampoules having a wall thickness of 0.2 mm. However, it will be appreciated that the principles of the present invention also may be applied to spherical or elongated polygonal ampoules. Furthermore, it will be appreciated that the principles of the present invention may be applied to one ampoule or more than two ampoules and to any size ampoule or vial. Furthermore, in one embodiment, no ampoules are used with the applicator.

Preferably, ampoules 14 and 15 are formed of glass, although other materials are entirely within the scope of the present invention. Other ampoules may include breakable plastic, foil or plastic film pouches. In one embodiment, the applicator does not contain ampoules, but contains solution within the body 12 that is retained by a seal, such as a foil seal. In the illustrated embodiment, ampoules 14 and 15 are placed side by side within body 12. The wall of glass ampoules 14 and 15 is of a thickness sufficient to contain the desired liquid during transport and storage, yet allow ampoules 14 and 15 to be fractured upon the application of localized pressure. In another embodiment, the ampoule consist of a flexible plastic or foil tube that is sealed on sealed on both ends and contains solution within the sealed tube. The ampoule is then cut or pierced to release the solution.

Body 12 is generally hollow and oval or elliptical in shape and includes axially opposed first and second ends 18, 20. Although it will be appreciated that body 12 may be a variety of shapes. The proximal first end 18 opens into the elongated head 24 and distal second end 20 is closed with cap 19. Illustrated body 12 and elongated head 24 are formed of high density polyethylene, although any material exhibiting similar flexibility and integrity may be used. In the preferred embodiment, second end 20 is closed with cap 19, however second end may also be closed during the molding process obviating the need for a cap or the like. The end of angled head 24 is closed with a tip cap 32, although the end of angled head 24 may also be closed during the molding process to obviate the need for a tip cap or the like. In the illustrated embodiment, body 12, elongated head 24, cap 19 and tip cap 32 were molded with 100% virgin material DOW, HDPE, Resin # 12454N, as defined in FDA Master File Number 4251.

Body 12 includes an interior wall 21 which defines an internal chamber 22 within body 12. Illustrated body 12 is elongated and defines a central longitudinal axis "x". Interior wall 21 is shaped to conform generally to the shape of ampoules 14 and 15 which are received within internal chamber 22. The circumference of interior wall 21 is slightly larger than the outer surface of the two ampoule bodies. In one embodiment, interior wall 21 is tapered to allow for the facilitate molding and for compressing the porous plug 46 along the interior wall 21 and support feature 17 to minimize glass fragments passing through the porous plug 46. In one embodiment, one side of the interior wall 21 is tapered at an angle of 0.225 degrees.

The thickness of the wall of the applicator may be between 0.040 to 0.080 inches (1-2 mm) and preferably is approximately 0.060 inches, except thin wall 40. The thickness of the wall of body 12 is reduced around crush area 42. Thin wall 40 may be between 0.020 to 0.040 (0.5-1 mm) inches and preferably is 0.030 inches. However, it will be appreciated that different wall sizes may be used within the scope of the embodiment of the invention. Thin wall 40 makes it easier for crush portion 36 of mechanism 26 to fracture ampoules when mechanism 26 is depressed.

Support feature 17 of hollow body 12 separates ampoules 14 and 15 and maintains ampoules 14 and 15 within internal chamber 22. With reference to FIG. 6, support feature 17 allows for ampoules 14 and 15 to be separated from one another to minimize breakage. In particular, support feature 17 prevents ampoules 14 and 15 from touching no matter the orientation of the applicator.

In this embodiment, support feature 17 is formed high-density polyethylene used to form body 12 and is integrally formed with body 12. Although it will be appreciated that support feature 17 may be made from any variety of materials and may be formed separate from body 12. It will be appreciated that support feature 17 may vary in length and height depending on the size of the applicator and ampoules.

In one embodiment, the support feature 17 is about 0.37 inches (9.4 mm) tall. In this embodiment, the height of the top portion of the support feature is 0.13 inches (3.3 mm) followed by about 0.09 inches (2.3 mm) of a 14.degree. taper on the lower portion of the support feature. It will be appreciated that support feature 17 may be tapered and have varying widths and height. For example, the width of the support feature 17 at the top is about 0.02 inches (0.5 mm) while the width of the support feature where it is molded with then body 12 is about 0.07 inches (1.8 mm).

With reference to FIG. 8, an alternative support feature 56 is shown. In this embodiment, the height of the support feature is about 0.23 inches (5.8 mm) and the width of the support feature 56 is about 0.08 inches (2.0 mm) Support feature 56 supports two 20 ml ampoules 14 and 15 retained within the body of the applicator. The body of the applicator depicted in FIG. 8 is about 1.30 inches (33 mm) wide and about 0.76 inches (19 mm) in height. The applicator depicted in FIG. 8 is about 13.64 inches (346 mm) long including the head and body. Ampoules 14 and 15 of FIG. 8 are approximately 8.5 inches (215 mm) in length. J

With further reference to FIG. 8, the interior of the body may contain one or more support ribs 58 for supporting ampoules 14 and 15 within the body. In the illustrated embodiment, the body includes three support ribs 58 for each ampoule. Support ribs 58 may be any number, length, height and width necessary to help support one or more ampoules within the body of the applicator. In the illustrated embodiment, the support ribs are 0.06 inches wide and run the length of the body of the applicator. In the illustrated embodiment, the support feature 56 and support ribs 58 abut ampoules 14 and 15. The support feature 56 and support ribs 58 help prevent breakage of ampoules 14 and 15 by keeping them in place during transport and handling of the applicator. It will be appreciated while support feature 56 and support ribs 58 are depicted as being used with an applicator having two 20 ml ampoules, support feature 56 and support ribs 58 may be utilized with any number or size of ampoules.

With reference again to FIG. 1, FIG. 2, FIG. 4 and FIG. 5, body 12 further presents an elongated head 24 protruding from body 12. Head 24 is generally hollow and oval or elliptical in shape. Although it will be appreciated that head 24 may be a variety of shapes. Head 24 includes an interior wall defining an internal chamber within head 24. When the ampoules are fractured, the liquid released from the ampoules flows into the interior chamber 22 of body 12, then into the interior chamber of head 24.

In one embodiment, head 24 is continuously molded to body 12 and is disposed at an angle. Preferably, head 24 is disposed an angle of about 15.degree., with respect to the central longitudinal axis of the body (x). It will be appreciated that head 24 may be disposed at a variety of angles with respect to the central longitudinal axis of body 12, preferable between 0-30.degree., and most preferably between 10-20.degree. with respect to the central longitudinal axis of the body (x). Head 24 is adapted to support porous element 16, as more fully described below.

In the illustrated embodiment, the head 24 is about 2.79 inches (71 mm) in length. However, it will be appreciated that the length of head 24 may vary depending on the size of the applicator. In some embodiments, the length of the head is about 0.5 inches (13 mm) to about 5 inches (127 mm) in length. The width of outside of head in illustrated embodiment is about 0.75 inches. The width of outside of body 12 is about 1.2 inches (30 mm). In this embodiment, the ratio of the width of the outside of the head to the body is 62.5%.

Head 24 has at least two openings to allow the liquid from the fractured ampoules to flow out of the head 24 and saturate porous element 16. With reference to FIG. 2, in one embodiment, two openings 30 are located on either side of head 24. In one embodiment, the openings are about 0.15 to 0.19 inches (3.8-4.8 mm) in diameter, preferable about 0.17 inches in diameter. It will be appreciated that the openings may be of any necessary size to allow liquid to flow from the interior chamber of the head 24 onto the porous element. In an alternative embodiment, only one opening 30 is located on each side of the head 24. In this embodiment, the two openings are each about 0.21 to 0.26 inches (5.3-6.6 mm) in diameter.

It will be appreciated that openings may be top and/or bottom portion of head 24 as well. The number of openings is based on the type and shape of the porous element and how the porous element is to be loaded with the liquid. In one embodiment, two openings are located on the top and bottom of head 24. In one embodiment the openings are about 0.15 to 0.19 inches in diameter, preferable about 0.17 inches in diameter. Although depicted as being round, it will be appreciated that openings 30 may be any shape that allows liquid to flow through. The openings on the elongated head allow for metered distribution of the liquid to the porous element. The porous element is filled from the rear and then forward allowing even distribution of the liquid through the porous element, when held in a vertical position, with respect to axis X.

In one embodiment, the head 24 is closed by tip cap 32 as shown in FIGS. 2 and 7. In this embodiment, tip cap 32 has at least one opening 34. In the exemplary embodiment, the diameter of the opening is about 0.04 to 0.07 inches (1-1.8 mm) in diameter, preferably 0.063 inches (1.6 mm) in diameter. However, it will be appreciated that the one or more openings 34 in tip cap 32 may be of any necessary size to allow liquid to flow from the head 24 onto the porous element.

Tip cap 32 allows closure of the molded elongated head 24, while allowing a rounded tip to minimize any abrasion to a patient. Tip cap opening 34 allows for the captured liquid volume in the elongated head to be used to wet the front tip of the porous element 16 and minimizes waste of the liquid from the ampoules. Thus, the at least two openings 30 in the elongated head 24 and the opening 34 in the tip cap 32 allow for uniform distribution of the liquid through the porous element 16 as the porous element has a larger surface area than the porous elements of typical applicators.

Porous element 16, such as a sponge or the like, covers head 24. As such, the porous element, unlike previous applicators, is not in direct contact with the body 12 of the applicator. Rather a more metered flow occurs when the liquid flows from the body through the head 24 of the applicator.

Porous element 16 may be formed of felt or an open-celled foam material. In the illustrated embodiment, porous element 16 was formed of a non-reticulated polyester polyurethane. In another embodiment, SIF-# 3-1000Z felt, (Natural Color Non-Pigmented) may be utilized. In yet another embodiment, Libero polyurethane foam obtained from Foamex International Inc. at 1000 Columbia Avenue Linwood, Pa. 19061 may be utilized. For example, reticulated polyester urethane or hydrophilic polyester polyurethane foam made by reacting one or more polyols with one or more isocyanates in the presence of a catalyst as described in U.S. patent application Ser. No. 11/353,816, may be utilized.

Porous element 16 is cut from a sheet of foam or felt material having the desired porosity for the liquid to be dispensed. Porous element 16 is preferably a shape that can cover head 24. For example, the foam may any shape of foam having a slit or opening cut therein to be placed over head 24 as shown in FIG. 3. It will be appreciated that the porous element 16 may be of any desired size and shape which is capable of significantly covering elongated head 24. For example, the shape of the foam may be any 3-dimensional shape that covers the elongated head 24. For instance, the foam may be in the shape of a sphere, cube, cone, prism, wedge-type 3-dimensional shape and combinations thereof. In the illustrated embodiment, the porous element 16 is a combination of a 3-dimensional rectangle and a 2-dimensional semi-circle.

In this embodiment, the porous element is 4 inches (102 mm) long, by 2 inches (51 mm) wide, by 1 inch (25 mm) tall. The 3-dimensional semi-circle has a radius of 1.00. An opening 37 is made at the mid-point of the height and width of the 3-dimensional rectangular portion of the porous element 16 as shown in FIG. 3. The opening 37 can be made in a variety of ways, including by a knife or blade cut. The blade cut in the present embodiment is about 2.75 inches (70 mm) in length about 0.82 inches (21 mm) wide.

The porous element 16 has two substantially wide flat sides, 50, 52 and a front tip 54. The multiple sides of the porous element 16 allow liquid to be applied to multiple surfaces and one time and for the applicator be used in difficult to reach spaces. In other words, flat side 50 of porous element may be able to apply a liquid, such as an antiseptic to one side of a skin fold while flat side 52 can apply antiseptic to the other side of the skin fold of a patient. It will be appreciated that although the porous element 16 is shown as having flat sides, the porous element may also have rounded sides.

A woven or non-woven laminate material may be laminated to porous element 16. The material laminate material may be a woven or non-woven polyester material. In one embodiment, Novonnete (SP-64 (3905) Polyester (Non-Woven) was laminated to 0.360".+-.0.032" (9.1 +- 0.81 mm) SIF-# 3-1000Z felt, (Natural Color Non-Pigmented) Reticulated Polyester Urethane. The laminate material is positioned between porous element 16 and head 24 of body 12. The laminate material provides a suitable welding material for securing the porous element in place on the head when an ultrasonic welding operation is used to manufacture the applicator. Alternatively, the head 24 and the porous element 16 may be bonded together with other suitable securing expedients. For example, porous element 16 could be secured to the head 24 by an adhesive or stitching or by head sealing or chemically bonding the element in place. Such alternative securing expedients are contemplated to be with the scope of the present invention.

In the illustrated embodiment, porous plug 46 is positioned between head 24 and ampoules 14 and 15. Porous plug 46 may be an open-celled foam material or felt. In the one embodiment, Novonette (SP-64 (3905) Polyester (Non-Woven) was laminated to 0.360".+-.0.032" SIF-#3-1000Z Felt, (Natural Color Non-Pigmented) Reticulated Polyester Urethane. Porous plug 46 helps control the rate liquid flows from the body and prevents shards of glass from pushing through porous element 16 during use of the applicator. Porous plug 46 is cut from a sheet of foam or felt material having the desired porosity for the liquid to be dispensed.

The body of the liquid applicator may also contain porous plug stops 44 for providing support for the porous plug 46 and helping to maximize flow of the liquid to the elongated head and porous element by maximizing internal flow volume within the body of the applicator. As shown in FIG. 5, the porous plug stops 44 are position between the porous plug 46 and the head 24. It will be appreciated that the porous plug stops 44 may be a variety of shapes and sizes depending on the size of the applicator and size of the porous plug 46. Furthermore, it will be appreciated that in some embodiments the liquid applicator may not include a porous plug 46 or porous plug stops 44.

Body 12 may also include at least one fracturing mechanism 26 projecting from the top of body 12. However, it will be appreciated that the at least one fracturing mechanism 26 may project from any portion of body 12. Mechanism 26 is any mechanism for fracturing more than one ampoule at substantially the same time. Mechanism 26, includes hinge portion 38, crush portion 36 and handling portion 34 extending from the distal end of mechanism 26. Preferably, mechanism 26 extends outwardly from body 12 at an angle of between 20.degree. and 40.degree. with respect to the central longitudinal axis of body 12. More preferably, mechanism 26 extends from body 12 at approximately 27.degree. with respect to the central longitudinal axis "x" of body 12. It will be appreciated that mechanism 26 may be disposed at a variety of angles with respect to the central longitudinal axis of body 12 and that more than one mechanism 26 may be utilized.

In the illustrated embodiment, mechanism 26 is continuously molded with body 12. It will be understood and appreciated, however, that separately formed mechanisms are contemplated to be within the scope of the present invention.

Handling portion 34 of mechanism 26 of the illustrated embodiment is spaced between 0.5 and 1.5 inches (13-38 mm) from body 12. Preferably, handling portion 34 is spaced approximately 1.0 inch from body 12. Handling portion 34 of mechanism 26 includes a textured outer surface to facilitate handling of applicator 10 and to inhibit slippage from the user's hand during application.

In the illustrated embodiment, mechanism 26 includes crush portion 36 and hinge portion 38 attached to body 12. It will be appreciated, however, that the principles of the present invention are equally applicable to various other structures for fracturing ampoules 14 and 15, such as multiple crush portions, multiple hinge portions and a crush portion that may be attached or detached to body 12. Handling portion 34 of mechanism 26 presents a gripping area which is significantly larger than the area of crush portion 36. Upon depression of mechanism 26, crush portion 36, flexes body 12 inwardly at thin wall 40, thereby localizing the forces effected by depressing mechanism 26 toward body 12 and enhancing fracturing of ampoules 14 and 15 as more fully described below.

Several features of mechanism 26 of the illustrated embodiment enhance the ability to fracture at least two ampoules at the same time including: the thickness of mechanism 26, the curvature of mechanism 26, the thickness of hinge portion 38 and the width of crush portion 36. The thickness of mechanism 26 is approximately 0.080 to 0.15 inches (2-4 mm) and preferably is 0.11 inches. In the illustrated embodiment, mechanism 26 is approximately 2.35 inches (51.7 mm) long. Hinge portion 38 of the illustrated embodiment is thinner than the rest of mechanism 26. Hinge portion 38 is approximately 0.040 to 0.080 inches thick, preferably 0.060 inches thick. The curvature of mechanism 26 increase the leverage of handling portion 34 of mechanism 26 making it easier for the user to fracture two ampoules substantially simultaneously.

The ratio of the width of crush portion 36 to the width of ampoules 14 and 15 side by side is important with respect to reliable breakage of ampoules 14 and 15. In the illustrated embodiment, the width of the crush portion 36 had to be at least approximately 1/5 the width of the two ampoules side by side to produce breakage of the ampoules almost simultaneously. The width of the two ampoules side by side was approximately 1.03 inches (26.2 mm) The minimum width of the crush portion of the mechanism that produces breakage of the ampoules almost simultaneously was 0.200 inches (5 mm). Thus, a length aspect ratio for reliably ampoule break was 1.03/0.200 or 5.15. All of these features, either singularly or in combination, along with thin wall 40, help enhance the ability of the mechanism to break multiple ampoules at the same time.

With reference to FIGS. 1 and 5, in use, applicator 10 presents a hand-held liquid applicator wherein mechanism 26 is depressed to release the desired liquid contained within ampoules 14 and 15 therein for application to a surface. Applicator 10 of the illustrated embodiment is grasped by one hand of a user. The bottom of body 12 is grasped with the palm and fingers of user, the user's fingers wrap around the bottom and side of the body 10 so the tips of the user's fingers rest on the top of body 12. The thumb of the same hand is positioned on handling portion 34 of mechanism 26 allowing for single-handed operation. The user depresses mechanism 26 toward body 12 to fracture ampoules 14 and 15. The movement of mechanism 26 is transferred by crush portion 38 to thin wall 40 of body 12 to deform body 12 inwardly and exert discrete localized fracturing forces against ampoules 14 and 15. Mechanism 26 provides an action that gains mechanical advantage as mechanism 26 is depressed toward body 12. Accordingly, if the user has limited gripping strength, or if the wall of the ampoule is exceptionally thick, the mechanism ensures fracturing of the ampoules.

Once mechanism 26 has been sufficiently depressed, the resulting forces fracture ampoules 14 and 15 almost simultaneously, thus releasing the liquid contained in each ampoule. Once ampoules 14 and 15 are fractured, the released liquid saturates porous plug element 46 which controls the rate of the flow and then the liquid saturates porous element 16. Consequently, body 12 essentially functions as a reservoir of the desired liquid. When the applicator is manipulated for scrubbing with the distal end oriented away from the surface to be scrubbed and the porous element oriented toward the surface as shown in FIG. 1, the liquid will flow from the fractured ampoules under the force of gravity down body 12, through porous plug 46 the through open end 18 and into head 24. The liquid flows through openings 30 of head 24 and through porous element 16. Thereafter, application of the liquid is accomplished by bringing porous element 16 into contact with the desired surface. The user may then use a painting or scrubbing motion to apply the liquid to the surface. The applicator may be used to apply liquid to hard to reach areas and multiple surfaces at one time.

During formation of the applicator, the molded applicator body is placed into a fixture to hold it in a particular orientation. The porous plug is inserted into the applicator body until it seats on the molded porous plug stops. Two 13 ml ampoules are inserted, and the end cap placed on the distal end of the applicator. The cap is seated by applying force to the cap while the body is held firmly. The tip cap can then be inserted to the open end of the head of the applicator. Typically a friction will hold the tip cap in-place, although a heat staking (or similar) process can also be utilized to insure that tip cap is retained. Once the tip cap is placed the porous element is bounded to the head of the applicator. The porous element can then be bonded in-place, by any of the previously mentioned methods.

Constructed and operated as previously described, this invention provides a hand-held liquid applicator of quality construction having a body with a mechanism that may be depressed toward the body to fracture at least two ampoules of liquid contained within the body. Further, this invention provides a disposable liquid applicator which permits single-handed operation in order to free the second hand of the user for use in assisting application of the liquid to the desired area. The liquid applicator of the present invention also has an elongated head that is completely covered by a porous element allowing the applicator to be used in hard to reach places.

From the foregoing, it will be seen that this invention is one well adapted to attain all the ends and objects hereinabove set forth together with other advantages which are obvious and which are inherent in the structure.

It will be understood that certain features and subcombinations are of utility and may be employed without reference to other features and subcombinations. This is contemplated by and is within the scope of the claims.

Since many possible embodiments may be made of the invention without departing from the scope thereof, it is to be understood that all matter herein set forth or shown in the accompanying drawings is to be interpreted as illustrative and not in a limiting sense.

## Claims

1. Liquid applicator (10) for applying a desired liquid to a surface, the applicator comprising:
- at least one ampoule (14,15) formed of a frangible material and containing liquid to be applied;
- an elongated hollow body (12), said body defining an internal chamber (22) adapted to receive said at least one ampoule (14,15);
- at least one mechanism (26) projecting from said body, said mechanism (26) flexing said body inwardly to fracture said at least one ampoule (14,15);
- a head (24) projecting at an angle from said body:
- a porous element (16) secured to said head (24);
and being **characterized in that**:
- the head (24) is an elongated head (24) _having at least two openings (30) for liquid to flow through;
- the porous element (16) is secured to said elongated head (24), in such a way that a portion of said elongated head is disposed within said porous element, and such that liquid flows through the at least two openings (30) of the elongated head (24) and through said porous element (16) when said ampoule (14,15) is fractured:
- wherein said at least two openings are provided on said portion of said elongated head that is disposed within said porous element.

2. Liquid applicator (10) according to claim 1, wherein the elongated head (24) has at least one side and the porous element (16) covers the at least one side of the elongated head (24).

3. Liquid applicator (10) according to claim 2, wherein the porous element (16) has two substantially wide flat (50,52) or rounded sides and a front tip (54).

4. Liquid applicator (10) according to any claim 1-3, further comprising: a support feature (17) positioned in between at least two ampoules (14,15), wherein the support feature (17) separates the two ampoules (14,15) from touching no matter the orientation of the applicator.

5. Liquid applicator (10) according to any claim 1-4, wherein the body (12) has a central longitudinal axis (x), the head (24) being preferably disposed at an angle of between 10 and 20 degrees with respect to the central longitudinal axis (x) of the body (12), more preferably at an angle of 15 degrees with respect to the central longitudinal axis (x) of the body (12).

6. Liquid applicator (10) according to any claim 1-5, further comprising a tip cap (32), the tip cap (32) optionally having at least one opening (34) allowing liquid to flow from the head (24) to the porous element (16), the opening (34) of the tip cap (32) being preferably between 0.04 and 0.07 inches in diameter.

7. Liquid applicator (10) according to any claim 1-6, further comprising two openings (30) on each side of the head to allow liquid from the head (24) to flow onto the porous element (16).

8. Liquid applicator (10) according to any claim 1-7, wherein the porous element (16) has at least two flat sides (50,52) capable of applying liquid to one or more surfaces.

9. Liquid applicator (10) according to any claim 1-8, wherein the porous element (16) is one of a foam or felt material.

10. Liquid applicator (10) according to any claim 1-9, further comprising: a support feature (56) for separating at least two ampoules (14,15), wherein the support feature (56) mechanically supports the at least two ampoules (14,15) to prevent breakage.

11. Liquid applicator (10) according to any claim 1-10, further comprising: two openings (30) on one of the top or bottom of the elongated head (24) to allow liquid from the head (24) to flow onto the porous element (16).

## Patentansprüche

1. Flüssigkeitsapplikator (10) zum Applizieren einer gewünschten Flüssigkeit zu einer Oberfläche, wobei der Applikator umfasst:
- mindestens eine Ampulle (14, 15), die aus einem zerbrechlichen Material gebildet ist und eine zu applizierende Flüssigkeit enthält;
- einen länglichen hohlen Körper (12), wobei der Körper eine innere Kammer (22) definiert, die angepasst ist, die mindestens eine Ampulle (14, 15) aufzunehmen;
- mindestens einen Mechanismus (26), der von dem Körper vorragt, wobei der Mechanismus (26) den Körper einwärts biegt, um die mindestens eine Ampulle (14, 15) zu brechen;
- einen Kopf (24), der mit einem Winkel von dem Körper vorragt;
- ein poröses Element (16), das an dem Kopf (24) befestigt ist;
und **dadurch gekennzeichnet ist, dass**:
- der Kopf (24) ein länglicher Kopf (24) ist, der mindestens zwei Öffnungen (30) für Flüssigkeit zum Durchfluss aufweist;
- das poröse Element (16) an dem länglichen Kopf (24) befestigt ist, in einer Weise, dass ein Teilbereich des länglichen Kopfes in dem porösen Element angeordnet ist, und dass Flüssigkeit durch die mindestens zwei Öffnungen (30) von dem länglichen Kopf (24) und durch das poröse Element (16) fließt, wenn die Ampulle (14, 15) gebrochen ist;
- wobei die mindestens zwei Öffnungen auf dem Teilbereich des länglichen Kopfes vorgesehen sind, der in dem porösen Element angeordnet ist.

2. Flüssigkeitsapplikator (10) gemäß Anspruch 1, wobei der längliche Kopf (24) mindestens eine Seite aufweist und das poröse Element (16) die mindestens eine Seite des länglichen Kopfes (24) bedeckt.

3. Flüssigkeitsapplikator (10) gemäß Anspruch 2, wobei das poröse Element (16) zwei im Wesentlichen weite flache (50, 52) oder gerundete Seiten und eine Frontspitze (54) aufweist.

4. Flüssigkeitsapplikator (10) gemäß einem der Ansprüche 1-3, ferner umfassend: ein Trägermerkmal (17), das zwischen mindestens zwei Ampullen (14, 15) angeordnet ist, wobei das Trägermerkmal (17) die zwei Ampullen (14, 15) ungeachtet der Orientierung des Applikators von einer Berührung trennt.

5. Flüssigkeitsapplikator (10) gemäß einem der Ansprüche 1-4, wobei der Körper (12) eine zentrale Längsachse (x) aufweist, wobei der Kopf (24) bevorzugt mit einem Winkel zwischen 10 und 20 Grad in Bezug auf die zentrale Längsachse (x) des Körpers (12), weiter bevorzugt mit einem Winkel von 15 Grad in Bezug auf die zentrale Längsachse (x) des Körpers (12), angeordnet ist.

6. Flüssigkeitsapplikator (10) gemäß einem der Ansprüche 1-5, ferner umfassend eine Spitzenkappe (32), wobei die Spitzenkappe (32) optional mindestens eine Öffnung (34) aufweist, die erlaubt, dass Flüssigkeit von dem Kopf (24) zu dem porösen Element (16) fließt, wobei die Öffnung (34) der Spitzenkappe (32) vorzugsweise einen Durchmesser zwischen 0,04 und 0,07 Zoll aufweist.

7. Flüssigkeitsapplikator (10) gemäß einem der Ansprüche 1-6, ferner umfassend zwei Öffnungen (30) auf jeder Seite des Kopfes, um zu erlauben, dass Flüssigkeit von dem Kopf (24) auf das poröse Element (16) fließt.

8. Flüssigkeitsapplikator (10) gemäß einem der Ansprüche 1-7, wobei das poröse Element (16) mindestens zwei flache Seiten (50, 52) aufweist, die geeignet sind, Flüssigkeit zu einer oder mehreren Oberflächen zu applizieren.

9. Flüssigkeitsapplikator (10) gemäß einem der Ansprüche 1-8, wobei das poröse Element (16) eines aus einem Schaum- oder Filzmaterial ist.

10. Flüssigkeitsapplikator (10) gemäß einem der Ansprüche 1-9, ferner umfassend: ein Trägermerkmal (56) zum Trennen von mindestens zwei Ampullen (14, 15), wobei das Trägermerkmal (56) die mindestens zwei Ampullen (14, 15) mechanisch stützt, um einen Bruch zu verhindern.

11. Flüssigkeitsapplikator (10) gemäß einem der Ansprüche 1-10, ferner umfassend: zwei Öffnungen (30) an einer der Oberseite oder Unterseite des länglichen Kopfes (24), um zu erlauben, dass Flüssigkeit von dem Kopf (24) auf das poröse Element (16) fließt.

## Revendications

1. Applicateur de liquide (10) pour appliquer un liquide désiré sur une surface, l'applicateur comprenant :
- au moins une ampoule (14, 15) constituée en un matériau cassable et contenant un liquide devant être appliqué ;
- un corps creux allongé (12), ledit corps définissant une chambre intérieure (22) adaptée de façon à recevoir ladite ampoule au nombre d'au moins une (14, 15) ;
- au moins un mécanisme (26) faisant saillie à partir dudit corps, ledit mécanisme (26) infléchissant ledit corps vers l'intérieur de façon à fracturer ladite ampoule au nombre d'au moins une (14, 15) ;
- une tête (24) faisant saillie selon un certain angle à partir dudit corps ;
- un élément poreux (16) fixé à ladite tête (24) ;
et étant **caractérisé en ce que** :
- la tête (24) est une tête allongée (24) comportant au moins deux ouvertures (30) pour qu'un liquide s'écoule à travers celles-ci ;
- l'élément poreux (16) est fixé à ladite tête allongée (24), de telle sorte qu'une partie de ladite tête allongée soit disposée à l'intérieur dudit élément poreux, et de telle sorte qu'un liquide s'écoule à travers les ouvertures au nombre d'au moins deux (30) de la tête allongée (24) et à travers ledit élément poreux (16) lorsque ladite ampoule (14, 15) est fracturée ;
- dans lequel lesdites ouvertures au nombre d'au moins deux sont disposées sur ladite partie de ladite tête allongée qui est disposée à l'intérieur dudit élément poreux.

2. Applicateur de liquide (10) selon la revendication 1, dans lequel la tête allongée (24) comporte au moins un côté et l'élément poreux (16) recouvre le côté au nombre d'au moins un de la tête allongée (24).

3. Applicateur de liquide (10) selon la revendication 2, dans lequel l'élément poreux (16) comporte deux côtés sensiblement larges plats (50, 52) ou arrondis et une pointe avant (54).

4. Applicateur de liquide (10) selon l'une quelconque des revendications 1 à 3, comprenant de plus : un élément de support (17) positionné entre au moins deux ampoules (14, 15), l'élément de support (17) séparant les deux ampoules (14, 15) de façon à les empêcher de se toucher quelle que soit l'orientation de l'applicateur.

5. Applicateur de liquide (10) selon l'une quelconque des revendications 1 à 4, dans lequel le corps (12) comporte un axe longitudinal central (x), la tête (24) étant de préférence disposée selon un angle compris entre 10 et 20 degrés par rapport à l'axe longitudinal central (x) du corps (12), et, de façon plus préférable, selon un angle de 15 degrés par rapport à l'axe longitudinal central (x) du corps (12).

6. Applicateur de liquide (10) selon l'une quelconque des revendications 1 à 5, comprenant de plus un capuchon de pointe (32), le capuchon de pointe (32) comportant de façon optionnelle au moins une ouverture (34) permettant à un liquide de s'écouler à partir de la tête (24) vers l'élément poreux (16), l'ouverture (34) du capuchon de pointe (32) ayant de préférence un diamètre compris entre 1,016 mm (0,04 pouce) et 1,778 mm (0,07 pouce).

7. Applicateur de liquide (10) selon l'une quelconque des revendications 1 à 6, comprenant de plus deux ouvertures (30) de chaque côté de la tête de façon à permettre à un liquide venant de la tête (24) de s'écouler sur l'élément poreux (16).

8. Applicateur de liquide (10) selon l'une quelconque des revendications 1 à 7, dans lequel l'élément poreux (16) comporte au moins deux côtés plats (50, 52) susceptibles d'appliquer un liquide sur une ou plusieurs surfaces.

9. Applicateur de liquide (10) selon l'une quelconque des revendications 1 à 8, dans lequel l'élément poreux (16) est l'un parmi un matériau en mousse ou en feutre.

10. Applicateur de liquide (10) selon l'une quelconque des revendications 1 à 9, comprenant de plus : un élément de support (56) pour séparer au moins deux ampoules (14, 15), l'élément de support (56) supportant mécaniquement les ampoules au nombre d'au moins deux (14, 15) de façon à empêcher une cassure.

11. Applicateur de liquide (10) selon l'une quelconque des revendications 1 à 10, comprenant de plus : deux ouvertures (30) sur l'un du dessus ou du dessous de la tête allongée (24) de façon à permettre à un liquide venant de la tête (24) de s'écouler sur l'élément poreux (16).
